# EUROPEAN PATENT APPLICATION

(11) **EP 2 323 057 A2**
(43) Date of publication of application: **18.05.2011**
(21) Application number: 10251778.6
(22) Date of filing: 08.10.2010
(51) Int. Cl.: G06F 19/00

(54) **Computer that weighs**

(30) Priority: 09.10.2009 US 250134 P
(71) Applicant: Maeir, Dan, Skokie, IL 60076 (US); Crandall, Richard L., Aspen, CO 81611 (US)
(72) Inventor: Maeir, Dan, Skokie, IL 60076 (US); Crandall, Richard L., Aspen, CO 81611 (US)
(74) Representative: Kenrick, Mark Lloyd

(57) **Abstract**

A system and method of using an adaptable interface for a medical device such as a scale. The scale includes a memory, a display, a processor, and a measurement component to measure one or more characteristics of a subject, such as height or weight. The adaptable interface includes a reconfigurable driver module that enables communication with different Electronic Health Record ("EHR") systems. The interface can download new drivers or update drivers to reconfigure the driver module. The reconfigured driver module is then configured to communicate with an EHR system according to the particular EHR system communication protocol. The adaptable interface is modular and includes one or more mounting adapters to enable coupling to adjustable interface mounting assembly. The adjustable interface mounting assembly is also operable to receive other modular interface units.

## Description

### RELATED APPLICATIONS

The present application claims priority to U.S. Provisional Patent Application Serial No. 61/250,134, filed October 9, 2009, the entire contents of which is hereby incorporated by reference.

### FIELD OF THE INVENTION

The present invention relates to scales for measuring characteristics, such as weight, height, etc., of a person or other object.

### SUMMARY

In one independent embodiment, a medical device is operable to measure a characteristic of a subject, the characteristic including one of a weight of a subject and a height of a subject. The device may generally include a memory; a display; a measurement component operable to determine a measured characteristic of a subject and to output measurement data indicative of the measured characteristic; and a processor coupled to the memory and to the display, the processor being operable to receive measurement data from the measurement component, output the measurement data to the display to cause the display to depict the measurement data, and store the measurement data in the memory, the processor including a reconfigurable driver module for communicating with external records databases, the reconfigurable driver module including a first driver, a second driver, a first communication configuration, and a second communication configuration, the first driver configuring the reconfigurable driver module into the first communication configuration to communicate with a first external records database, the second driver configuring the reconfigurable driver module into the second communication configuration to communicate with a second external records database, the second external records database having a different communication protocol than the first external records database.

In another independent embodiment, a method of communication between a medical device and remote databases is provided. The medical device may include a measurement component and a processor. The method may generally include receiving, by the processor, measurement data from the measurement component, the measurement data indicating at least one of a weight of a subject and a height of a subject; receiving a first driver and a second driver by the processor from one or more remote devices over a network, the first driver defining a first communications protocol for communicating with a first remote database and the second driver defining a second communications protocol for communicating with a second remote database; receiving a command to store the measurement data in the first remote database; and communicating the measurement data to the first remote database for storage using the first driver.

In yet another independent embodiment, a method of communication between a medical device and remote databases may generally include receiving, by the processor, measurement data from the measurement component, the measurement data indicating at least one of a weight of a subject and a height of a subject; receiving a command to store the measurement data in a first remote database; determining that a first driver is an appropriate driver for communicating with the first remote database; determining that the processor does not include the first driver; obtaining the first driver from one or more remote devices over a network; communicating the measurement data to the first remote database for storage using the first driver.

In a further independent embodiment, a method of assembling a medical device may be provided. The method may generally include providing a frame having a lower portion and an upper portion, a measurement component coupled to the frame and operable to determine one of a weight of a subject and a height of a subject, the measurement component being operable to output a signal representative of the one of the determined weight and the determined height, and an adjustable interface mounting assembly coupled to the upper portion of the frame; providing a first interface head including a first display, a first input/output port for selectively coupling the first interface head to the measurement component, and a first mounting adapter for selectively coupling the first interface head to the adjustable interface mounting assembly, the first interface head being operable to receive measurement data from the measurement component and to display a value corresponding to the received measurement data; providing an adaptable, second interface head including a second display, a second input/output port for selectively coupling the second interface head to the measurement component, and a second mounting adapter for selectively coupling the second adjustable interface head to the adjustable interface mounting assembly, the second adjustable interface head being operable to receive measurement data from the measurement component and to display a value corresponding to the received measurement data, a memory, and a processor coupled to the memory and to the second display and including a reconfigurable driver module for communicating with external records databases, the reconfigurable driver module including a first driver, a second driver, a first communication configuration, and a second communication configuration, the first driver configuring the reconfigurable driver module into the first configuration to communicate with a first external records database and the second driver configuring the reconfigurable driver module into the second configuration to communicate with a second external records database, the second external records database having a different communication protocol than the first external records database; selecting one of the first interface head and the second interface head; and coupling the one of the first interface head and the second interface head to the measurement component and to the adjustable interface mounting assembly.

In still another independent embodiment, an adaptable head assembly for a medical device may be provided. The medical device may include a frame having a lower portion and an upper portion, a measurement component coupled to the frame and operable to determine one of a weight of a subject and a height of a subject, the measurement component being operable to output a signal representative of the one of the determined weight and the determined height, and an adjustable interface mounting assembly coupled to the upper portion of the frame. The head assembly may generally include a housing including a mounting portion connectable to the mounting assembly to support the head on the frame; a memory supported by the housing; a display supported by the housing, the housing being adjustably supportable on the frame to adjust a position of the display; an input/output port for selectively coupling the head assembly to the measurement component; and a processor coupled to the memory, to the display and to the input/output port, the processor being operable to receive measurement data from the measurement component, output the measurement data to the display to cause the display to depict the measurement data, and store the measurement data in the memory, the processor including a reconfigurable driver module for communicating with external records databases, the reconfigurable driver module including a first driver, a second driver, a first communication configuration, and a second communication configuration, the first driver configuring the reconfigurable driver module into the first communication configuration to communicate with a first external records database, the second driver configuring the reconfigurable driver module into the second communication configuration to communicate with a second external records database, the second external records database having a different communication protocol than the first external records database, the reconfigurable driver module being operable to obtain at least one of the first driver and the second driver from a remote device over a network.

Other independent aspects and independent features/advantages of the invention will become apparent by consideration of the detailed description, claims and accompanying drawings.

### BRIEF DESCRIPTION OF THE DRAWINGS

Fig. 1 is a block diagram of a system with a computer that weighs/measures ("CTW").

Fig. 2 is a block diagram of a CTW.

Figs. 3A-3B illustrate an exemplary process for using a CTW.

Fig. 4 illustrates an exemplary process for retrieving an external driver.

Fig. 5 illustrates a block diagram of a CTW interacting with an electronic health records system.

Figs. 6-9 illustrate various views of a CTW.

Figs. 10-21 illustrate an adjustable interface mounting assembly for the CTW.

Figs. 22-32 illustrate components usable with an adjustable interface mounting assembly and an interface for a scale assembly.

Figs. 33-35 illustrate an interface for a scale assembly.

Figs. 36-38 illustrate an adaptable interface for a scale assembly.

Figs. 39-41 illustrate an adaptable interface mounted to a scale assembly.

### DETAILED DESCRIPTION

Before any independent embodiments of the invention are explained in detail, it is to be understood that the invention is not limited in its application to the details of construction and the arrangement of components set forth in the following description or illustrated in the following drawings. The invention is capable of other independent embodiments and of being practiced or of being carried out in various ways.

As should also be apparent, the systems shown in the figures are models and/or examples of what actual systems might be like. Many of the modules and logical structures described are capable of being implemented in software executed by a microprocessor or a similar device or of being implemented in hardware using a variety of components including, for example, application specific integrated circuits ("ASICs"). Terms like "controller" and "module" may include or refer to both hardware and/or software. Furthermore, throughout the specification capitalized terms are used. Such terms are used to conform to common practices and to help correlate the description with the coding examples, equations, and/or drawings. However, no specific meaning is implied or should be inferred simply due to the use of capitalization. Thus, the claims should not be limited to the specific examples or terminology or to any specific hardware or software implementation or combination of software or hardware.

Height, weight and other measured characteristics of a person can be used by dieticians, medical doctors, nurses, and other personnel to monitor a person's fitness or health. Individuals and health care providers may record such measurements to track the information over time. The measured height and weight records may be manually entered into a paper record system or entered using a keyboard or other input device into an electronic record system.

Multiple electronic health records ("EHR") systems are used by various organizations, for instance, health care providing organizations. Exemplary EHR systems include Health Vault™, NextGen™, AllScripts™, eClinicalWorks™, and other health records systems, such as long term care records databases and past health history databases. An EHR system is populated with medical data and other patient information for numerous reasons, including monitoring the health of a patient. A patient's height and weight are exemplary data stored in the patient's medical record. In some instances, government regulations may encourage or require reporting of certain medical data (e.g., height and weight data) to EHRs or similar medical databases.

EHR systems may have unique communication interface protocols. A computer that is configured to communicate with a first EHR system using a first protocol cannot communicate with a second EHR system using the first protocol. Rather, the second EHR system communicates using a second protocol. Independent embodiments of this invention contemplate a measurement device (e.g., measuring weight, height, and/or other characteristics) that can be reconfigured to communicate with different EHR systems.

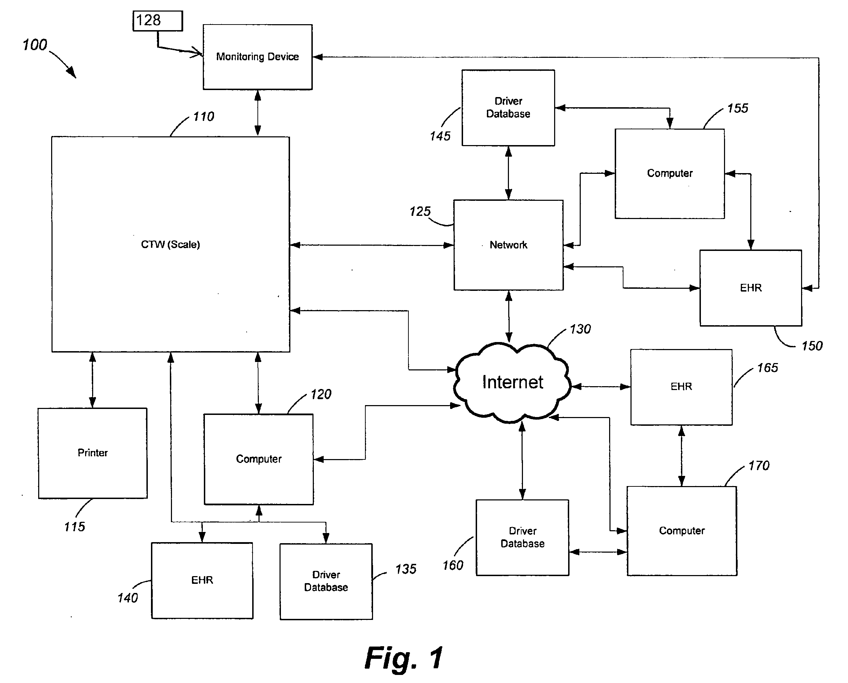

Fig. 1 depicts one exemplary embodiment of a system 100 with a Computer that Weighs ("CTW") 110. The illustrated CTW 110 is connected to a printer 115, a computer 120 (e.g., a desktop or laptop computer), a network 125, a monitoring device 128, and the Internet 130. The CTW 110 communicates with a driver database 135 and an EHR system 140 either directly or via the computer 120. The CTW 110 communicates with a driver database 145 and an EHR system 150 via the network 125 or via both the network 125 and a computer 155. The network 125 can be any suitable computer network, including a local area network ("LAN") and a wide area network ("WAN"). The CTW 110 communicates with a driver database 160 and an EHR system 165 via the Internet 130 or via both the Internet 130 and a computer 170.

The CTW 110 connects to the network 125, the Internet 130, the computer 120, the printer 115, the monitoring device 128, the driver database 135, and the EHR system 140 through a variety of interfaces. For example, the CTW 110 can connect using hard-wired connectors such as USB, RS-232, serial or parallel link, and Ethernet cables, or using wireless interfaces such as Bluetooth or IEEE 801.11 compatible devices. Other interfaces should also be apparent.

Furthermore, although the driver databases and the EHR system databases of Fig. 1 are shown external to the computers 120, 155 and 170, the databases may be internal or a combination or external and internal. The driver databases and the EHR system databases may be implemented using a combination of software (e.g., database management software) and hardware (e.g., processor and memory) as would be apparent. A driver database at least includes one or more drivers. A driver, also referred to as a specific interface program, can be considered a translator that enables a first device or program to communicate with a second device or program. In another sense, a driver can be considered software that controls the digital commands and data interactions between two devices or programs, often unique to one or both of the devices or programs. For instance, a driver enables a processor to communicate using a specific communication protocol of an external database or device such that the external database or device is operable to understand communications received from the processor and vice versa. In some independent embodiments, some or all of the driver databases are accessible via a web browser running on the CTW 110.

The monitoring device 128 monitors one or more patient characteristics or "vitals" such as, for example, heart rate, blood pressure, oxygen levels, etc. The monitoring device 128 receives height and/or weight information from the CTW 110. The monitoring device 128 is able to store the information locally, display the information on a local display screen, and provide the information to the EHR 150 for storage. In some independent embodiments, the monitoring device 128 outputs the patient characteristics obtained by the monitoring device 128 to the CTW 110. In turn, the CTW 110 is operable to store the characteristics locally, display the characteristics on a local display, and provide the information to an EHR system (e.g., EHR 140, 150 or 165).

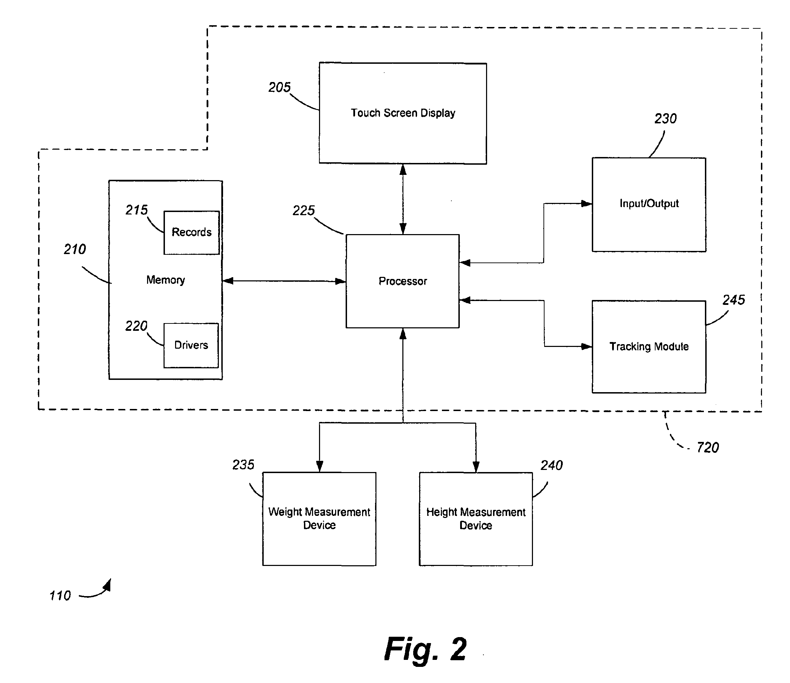

Fig. 2 illustrates one independent embodiment of the CTW 110. The illustrated CTW 110 includes a touch screen display 205, a memory 210 with stored records 215 and drivers 220, a processor 225, an input/output module 230, a weight measurement device 235, and a height measurement device 240. Although the block diagram depicts each component as having its own connection to the processor, a shared bus for some or all of the components may be used in other embodiments.

The touch screen display 205 enables a user to enter data into the CTW 110 and enables the CTW 110 to display data to the user. For example, the user may enter identifying information, such as a name, birth date, etc., using a displayed virtual keyboard, may navigate the World Wide Web by interacting with a displayed Internet browser, or may interact with any other software provided on the CTW 110 using the display 205. The display 205 will also show the user the current height, weight and/or other data being measured and display data retrieved from memory 210. In other embodiments, a keyboard, computer mouse, or other computer input device may be used to interact with the CTW 110.

The memory 210 provides storage capabilities to the processor 225. Among other data, the memory 210 stores records data 215 and drivers 220. The records data 215 includes measurements of weight, height, other characteristic(s) as well as data to identify the associated patient or user. The drivers 220 include driver software used by the CTW 110 to interact with EHR systems, as will be described in more detail below.

The weight measurement device 235 and the height measurement device 240 measure the weight and height, respectively, of a subject of the CTW 110. In some embodiments, only the weight measurement device 235 or, alternatively, only the height measurement device 240 is included in the CTW 110. In some embodiments, the CTW 110 receives height and weight measurements and calculates a body mass index ("BMI") of the subject. At least one of the weight, height, and BMI of a subject may be printed by the printer 115 and/or shown on the display 205, along with patient identifying information. Components of the CTW 110, such as the weight measurement device 235, the display 205, etc., may be similar to components shown in U.S. Patent No. 7,550,682, issued June 23, 2009, the entire contents of which are hereby incorporated by reference. In other embodiments, other devices are included in the CTW 110 to measure/determine other characteristic(s), such as, for example, heart rate, blood pressure, time and date of measurement(s), etc.

In some independent embodiments, the CTW 110 includes a tracking module 245. The tracking module 245 can track the weight (or other characteristic) of a user over time and compute first and second derivatives to determine the velocity and acceleration of weight loss/gain, respectively. The tracking module 245 may also include predetermined levels of velocity and acceleration. The tracking module 245 may then compare the calculated velocity and acceleration of weight loss/gain against the predetermined levels of velocity and acceleration.

The predetermined levels act as thresholds. When a threshold predetermined level is crossed, the tracking module can output an alert or message so that corrective action can be taken. Predetermined levels can be selected to ensure that a user's weight loss or gain is within a healthy range. For instance, if the rate of weight loss is not high enough (it does not cross a first predetermined level), the tracking module 245 can alert the user or a caretaker. Additionally, if the rate of weight loss is too great (it crosses both the first predetermined level and a second (higher) predetermined level), the tracking module 245 can alert the user or caretaker. The tracking module 245 can also output a message indicating that the rate of weight loss is appropriate if it crosses the first predetermined level, but not a second predetermined level. The output messages and alerts are, for instance, received by the processor 225 that shows them on the display 205, generates an audible message, sends them as a text message to a phone, and/or sends them as an e-mail.

Various other predetermined levels may be used to provide a finer granularity of feedback to a user or a caretaker. A similar calculation and comparison of predetermined levels of acceleration and calculated levels of acceleration can also be performed for other characteristics. Additionally, the messages and alerts, as well as the calculated velocities and accelerations, generated by the tracking module 245 can be stored as a record in an EHR.

In some independent embodiments, the tracking module may be a program stored in memory 210 or within memory (not shown) of the processor 225 on the CTW 110. The tracking module program may then be executed on the processor 225. In other independent embodiments, the tracking module is a separate hardware component or a combination of hardware and software that is within the CTW 110, external to the CTW 110, or a combination thereof.

The input/output module 230 enables the CTW 110 to communicate with external devices and EHR system databases as depicted in Fig. 1. The input/output module 230 includes one or more USB ports, LAN ports, serial RS-232 ports, a WiFi wireless adapter, and a Bluetooth adapter. Although the input/output 230 and the processor 225 are depicted as separate components of the CTW 110, in some embodiments, they may be combined into one module. The processor 225 is coupled to and communicates with the memory 210, the display 205, the input/output 230, and the measurement devices 235 and 240.

The CTW 110 also includes a power module (not shown) for receiving power and converting power for delivery to the other components of the CTW 110. The power source that provides power to the power module is any of a standard wall outlet providing 110 or 220 V AC power, one or more batteries providing DC power, or another power source. The CTW 110 also includes a power on/off button for selectively turning on and off the CTW 110.

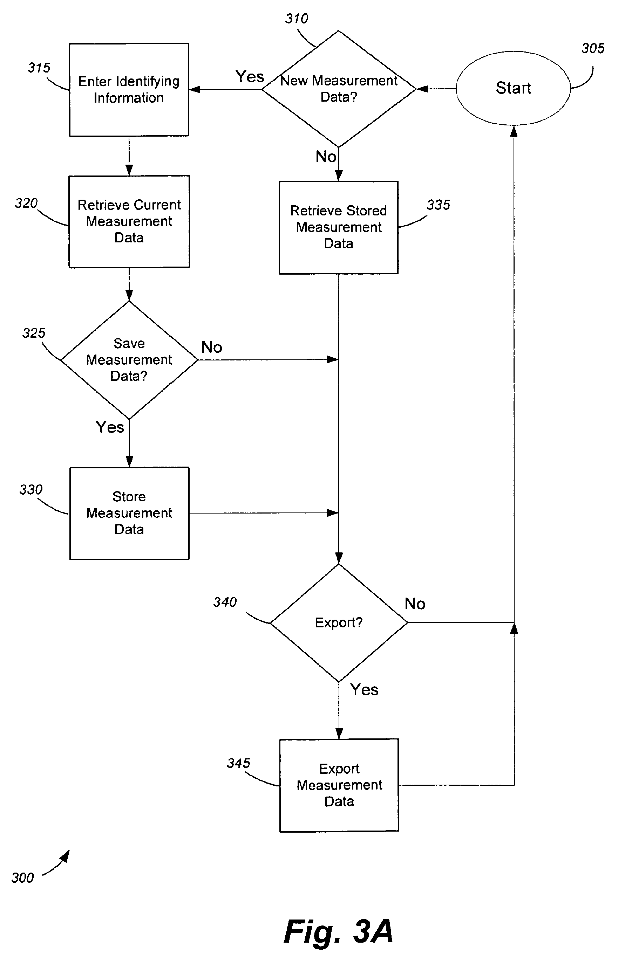

Fig. 3A depicts a method 300 implemented with the CTW 110. The method 300 starts in step 305. In step 310, a user decides whether to seek new measurement data or to retrieve measurement data from memory. If in step 310, the user decides to retrieve stored data, the method proceeds to step 335. In step 335, measurement data is retrieved from a memory (e.g., the memory 210). The measurement data is retrieved by using, for instance, a patient's name, birth date, or other identifying data. The retrieved data may be displayed on the display 205, and then the method proceeds to step 340 (described below).

If new measurement data is selected in step 310, the user enters identifying information in step 315. For instance, the name or patient number of the subject to be measured may be entered in the CTW 110 via display 205. In step 320, the CTW 110 measures the height, weight, other characteristic(s) of the subject. In step 325, the CTW 110 determines, e.g., based on user input, whether to save the measurement data and the identifying data in records 215. If the CTW 110 determines to store the data, the data is stored in step 330. After storing data in step 330 or determining not to store data in step 325, the method 300 proceeds to step 340.

In step 340, the CTW 110 determines whether to export the measurement data to an EHR system. If the CTW 110 determines not to export (e.g., the user chooses not to export data), the method 300 returns to the start in step 305. Otherwise, the method proceeds to step 345 to export the measurement data to an EHR system. As stated above, EHR systems may have unique communication interface protocols. For instance, EHR system 140, EHR system 150, and EHR system 165 each have unique protocols. To communicate with each EHR system, the CTW 110 uses a different driver to properly format communication data sent to the EHR system and received from the EHR system.

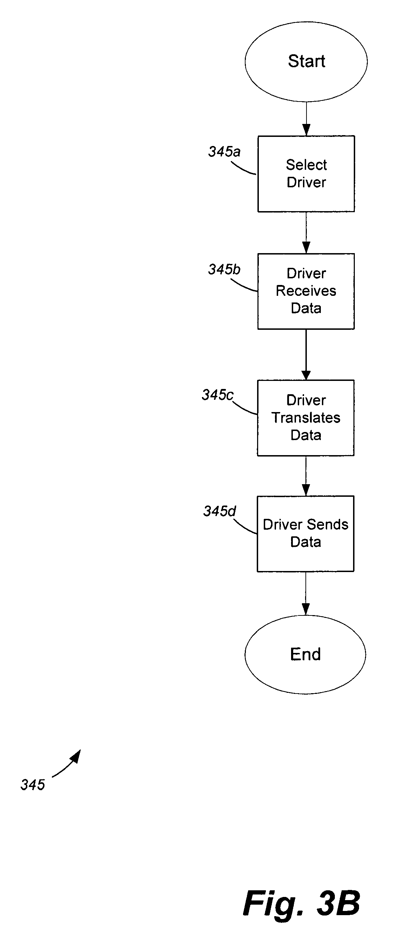

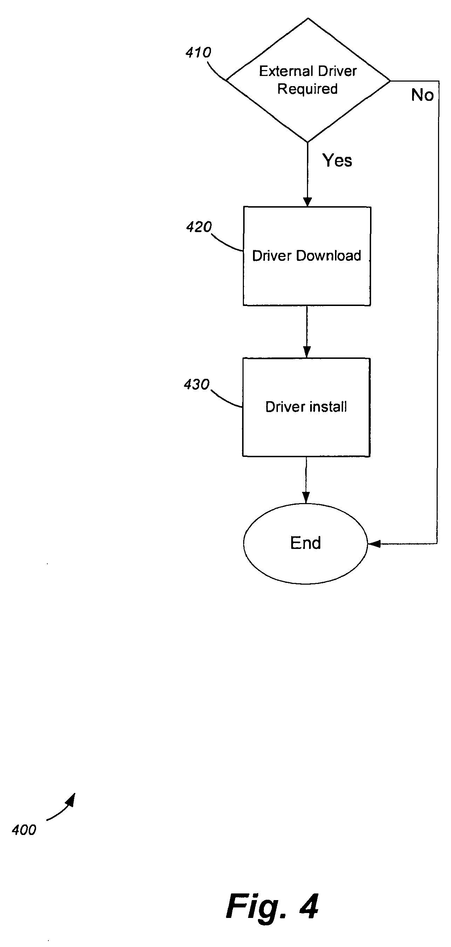

Exporting data to an EHR system (step 345) is depicted in Fig. 3B and includes the steps of selecting a driver (step 345*a*), the driver receiving the data (step 345b), the driver translating the data (step 345c), and the driver sending the data to the EHR system (step 345d). In step 345a, the CTW 110 selects the appropriate driver for use with the intended EHR system recipient database. The selected driver may already be installed on the CTW 110. In other situations, the driver may be stored in the memory 210 (i.e., one of the drivers 220) and may need to be installed on the CTW 110 upon selection. In other situations, the driver for communicating with the intended EHR system recipient database is stored external to the CTW 110. Driver selection will be described in more detail with respect to Fig. 4 below.

Once the appropriate driver is selected and, if necessary, installed, the data to be sent to the EHR system is forwarded to a driver in step 345b. The driver receives the data to be sent and an instruction to send (step 345b). The driver translates the instruction and data in accordance with the recipient EHR system protocols (step 345c). In some instances, the CTW 110 data format will match the EHR system data format, and no data formatting will be necessary. The driver module then communicates with the recipient EHR system according to the recipient EHR system communication protocols to send the data to the recipient EHR system (step 345d). In some embodiments, steps 345c and 345d are repeated one or more times to send all of the data to the EHR system.

In some embodiments, selecting a driver (step 345*a*) includes retrieval of an externally stored driver. Fig. 4 depicts one exemplary method 400 for retrieving an external driver. In step 410, the CTW 110 or a user determines whether an external driver is required. For instance, a user is able to select an EHR to which the data is to be exported. In response, the CTW 110 searches the drivers 220 in memory 210 for the selected EHR system's driver. If the driver is found, the CTW 110 proceeds to the end of method 400. If the driver is not found, an external driver is required, and the CTW 110 downloads the appropriate driver in step 420. The user may use a CTW 110 web browser using the touch screen display 205 or other input devices to find the appropriate driver to download. The driver may be available from a driver database (e.g., 135, 145 or 160) or from a website, for instance, a website associated with the particular EHR system.

In some embodiments, the CTW 110 automatically connects to a driver database (e.g., 135, 145 or 160) or other external source and downloads the appropriate EHR system driver. In some embodiments, the CTW 110 automatically sends a request to a driver database (e.g., 135, 145 or 160) or other external source and, in response, receives the appropriate EHR system driver. In some embodiments, an external device can initiate a new driver download. For instance, the external device can connect with the CTW 110 and command it to retrieve a particular driver, or the external device can simply send a particular driver to the CTW 110. An exemplary external device may be a computer controlled by an employee of a company associated with a particular EHR system or a computer controlled by an employee of the CTW 110 retailer or manufacturer.

Although the method 400 for retrieval of an external driver is described with reference to step 345a of Fig. 3B, the method 400 may be carried out at other times by a user or the CTW 110. For instance, a user may be aware that a particular EHR system driver (not stored within the CTW 110) is needed during an initial set-up of the CTW 110. The user may perform the method 400 to acquire the EHR system driver at this point, or any other point before being prompted by the CTW 110 during a data export operation. Additionally, the CTW 110 may automatically download predetermined drivers upon being powered for the first time. In other instances, the CTW 110 may update drivers already stored in memory 210 or installed on the CTW 110 at regularly scheduled intervals or upon request (e.g., from an external device or a user).

After downloading the appropriate driver in step 420, the driver is installed on the CTW 110 in step 430. The installed driver reconfigures a driver module on the CTW 110. In some embodiments, the driver module is a software program(s) run by an operating system of the CTW 110. That is, the driver module is software executed by the processor 225. In some instances, the driver module is a hardware input/output module (e.g., input/output module 230) that is configured by the installed driver to have the appropriate functions in accordance with an EHR system communications protocol.

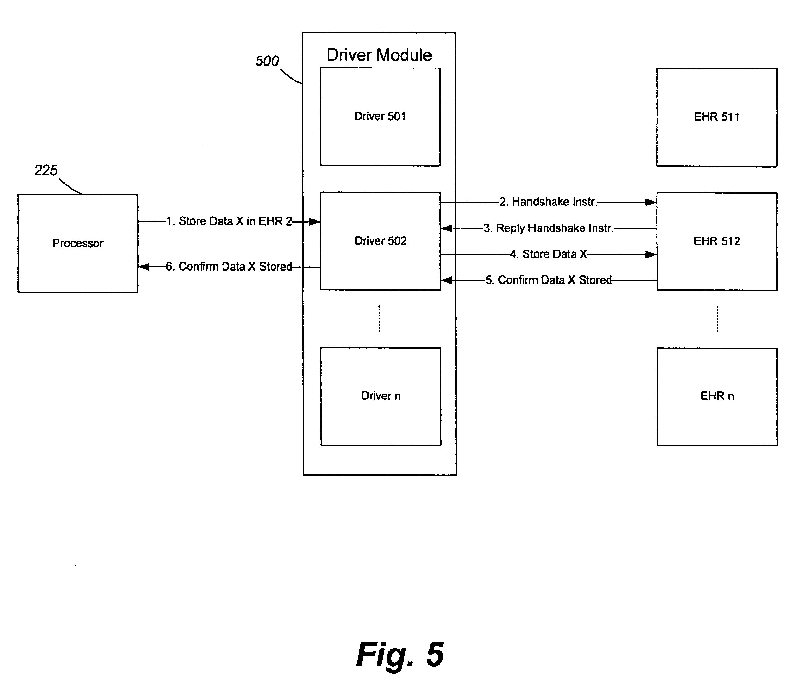

Fig. 5 depicts the processor 225, *n* separate drivers that make up a driver module 500, and *n* EHR system databases. Fig. 5 shows an exemplary and simplified communication of one independent embodiment. The communication includes the CTW 110 driver 502 receiving a "Store Data *x* to EHR 512" command from the processor 225. The driver 502 translates the command to a series of output signals or instructions to effect the store command. The translated command first includes a handshake instruction from the driver 502 to the EHR system 512. The EHR system 512 responds indicating a communication link has been established. Then, the driver 502 sends a store data *x* instruction to EHR system 512. The EHR system 512 stores data *x* in response to the received instruction. The EHR system 512 sends a confirmation signal to the driver 502 to indicate that the data *x* was successfully stored. Finally, the driver 502 sends a confirmation signal to the processor 225 to indicate that the data *x* was successfully stored in EHR system 512.

In another instance, processor 225 instructs the driver module 500 to store data *x* into EHR 511. The driver module 500 forwards the "Store Data *x* to EHR 511" command to the driver 501. The EHR 511 communicates without handshaking. As such, the driver 501 simply translates the store command to conform to the EHR 511 protocol and awaits a confirmation message from the EHR 511. The confirmation message is then forwarded from the driver 501 to the processor 225. In some instances, confirmation messages are not supplied by an EHR system to the driver module 500 or the processor 225.

Although shown external to the processor 225, in some embodiments, the driver module 500 may be partially or entirely within processor 225. Furthermore, although the driver module 500 is depicted with *n* separate drivers, the driver module 500 may include any number of active and inactive drivers and may have drivers added (e.g., see Fig. 4) and deleted over time. Active drivers can include those drivers currently running on the processor (e.g., as software by an operating system), for instance. Active drivers can also include those drivers that are currently implemented via hardware or a combination of software and hardware on the CTW 110 (either within or external to the processor 225). Inactive drivers include drivers that are stored locally on the CTW 110 (e.g., in the memory 210 or the processor 225), but that are not considered active drivers.

The CTW 110 is also operable to perform diagnostic testing and troubleshooting based either on local user control and/or remote control. For instance, a user selects, via the display 205, diagnostic testing and troubleshooting software (diagnostic software) stored in the memory 210 for execution on the processor 225. A user is also able to navigate using a web browser to locate and download diagnostic software stored remotely (e.g., on a manufacturer's or device support website). In other instances, a remotely located technician using one of the computers 120, 155 or 170 connects to and communicates with the CTW 110 to perform diagnostic testing and troubleshooting. For instance, the remote technician selected diagnostic software stored on the CTW 110 for execution, installs diagnostic software on the CTW 110 for execution, or executes diagnostic software at the remote site that communicates with the CTW 110 to investigate and potentially fix problems with the CTW 110.

The diagnostic software communicates with various components of the CTW 110 to determine whether they are properly functioning. For instance, the diagnostic software pings each sensor or load cell or other control unit in the weight measurement device 235 and height measurement device and awaits a confirmation from each component to verify proper operation. The diagnostic software also includes memory testing software to test proper functioning of the memory 210 and display testing software to test proper functioning of the display 205. The diagnostic software also includes the battery testing software to ensure proper functioning of battery power sources within the CTW 110.

Additionally, the CTW 110 is operable to receive new software or software updates/modifications from a local and/or remote technician. In some instances, whether due to market trends, government regulations, or otherwise, a user will desire new patient data and/or analysis, reporting software, etc. The CTW 110 receives updated or new software via, for instance, a computer connected directly, over a network, or over the Internet (see, e.g., various connections of Fig. 1). The reconfigurable software enables the CTW 110 to adapt to new regulations and market trends for different methods of determining, analyzing and/or reporting patient data.

For instance, a new patient metric involving a height/weight ratio other than BMI may be developed after the CTW 110 is already being used in the field (e.g., at a clinic or other health care facility). The CTW 110 can receive, from a technician at a remote computer over the Internet, new software to determine, compute, display, store, and/or report the new metric. Thus, the reconfigurable CTW 110 enables in-field updating/modification to adapt to new EHR database communication protocols, new patient data metrics and algorithms, government regulation requirements, etc.

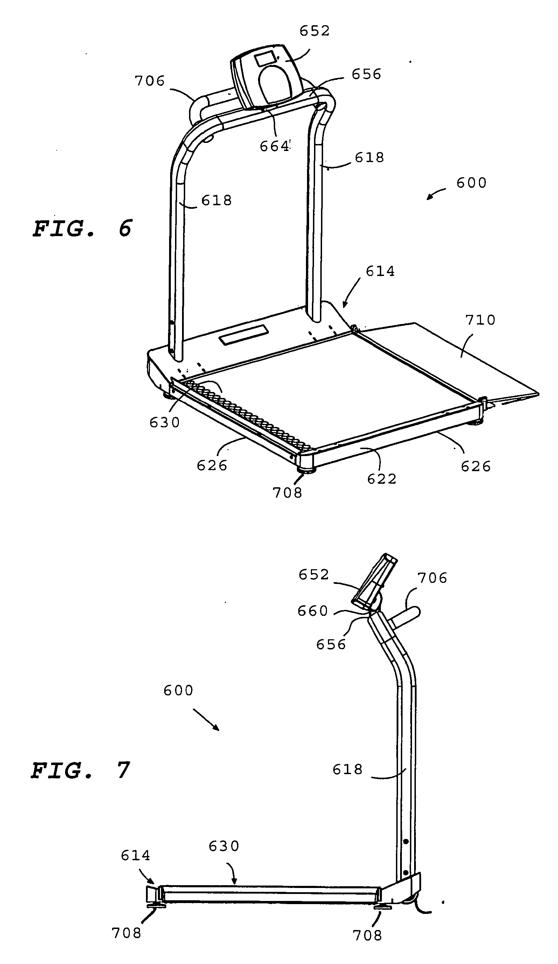

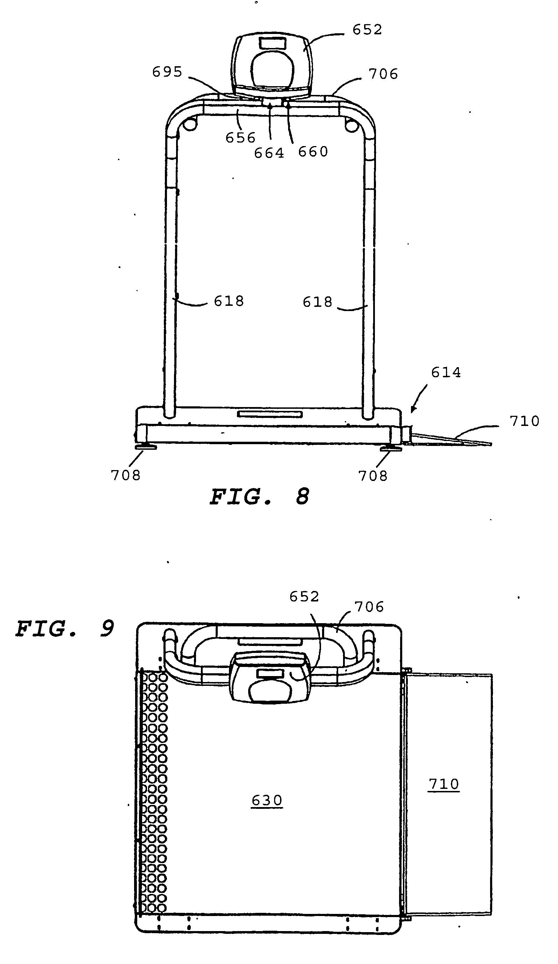

Figs. 6-9 depict an exemplary scale assembly 600 including a base 614 and pillars or one or more uprights 618 coupled to and extending from the base 614. The base 614 has a frame 622 having a plurality of walls 626 and a top 630. The base 614 includes load cells and is represented in Fig. 2 as the weight measurement device 235. The height measurement device 240 is not depicted in Figs. 6-9.

As illustrated in Figs. 6-9, the pillar 618 has two, spaced apart uprights that extend upwardly from the base to support a user interface 652. The pillars 618 can be two separate members or one bent member, in which both ends of the bent member connect to the base 614. The spaced apart configuration of the pillars 618 can be advantageous in some situations, such as, for example, when a seated person must be weighed. This allows a wheelchair occupant to properly position themselves on the scale without hitting the pillars 618 or having a centered pillar located between their legs. Also, the occupant may be better positioned to read the indicator. As illustrated, the pillars 618 of the illustrated embodiment are also bent towards the position of a person being weighed. Thus, the surface 656 that supports the interface 652 is some what cantilevered. This allows the interface 652 to be positioned closer to the person being weighed. Thus, the person does not have to reach as far to view and interact with the interface 652.

In some embodiments, however, the two, spaced apart pillars 618 located off-center are replaced or supplemented with one or more centered pillars. In some of these embodiments, the centered pillars support the user interface 652.

In some embodiments, such as the one illustrated, the scale assembly 600 is portable. As illustrated in Fig. 7, a wheel 704 is connected to the base 614. Another wheel (not shown) is coupled to the other side of the base 614. The scale assembly 600 merely needs to be tilted forward to move the scale assembly 600 by placing it on the wheels. Typically, one could grab the pillars 618 or a handle 706 coupled to the pillars 618 to tilt the scale assembly 600 onto the wheels 704. As illustrated in Fig. 7, the wheel 704 of the illustrated embodiment generally does not touch the ground when the scale assembly 600 is in an operating position. A plurality of supports or feet 708 are designed to support the scale assembly 600 with the wheels, including wheel 704, above the ground. However, in other embodiments, one or more sets of wheels can be in contact with the ground while in operation. These embodiments can have other features to prevent the scale from moving such as, for example, wheel locks, spring biased supports for the wheels, and the like. In some embodiments, the scale assembly 600 includes a ramp 710 for easing access for patients in wheelchairs or loading and unloading objects on wheels.

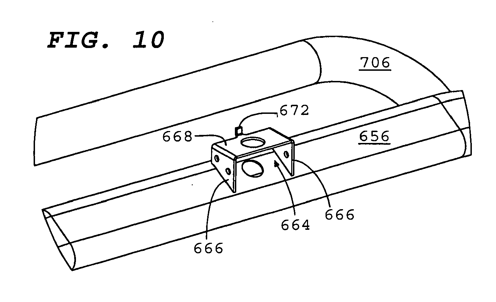

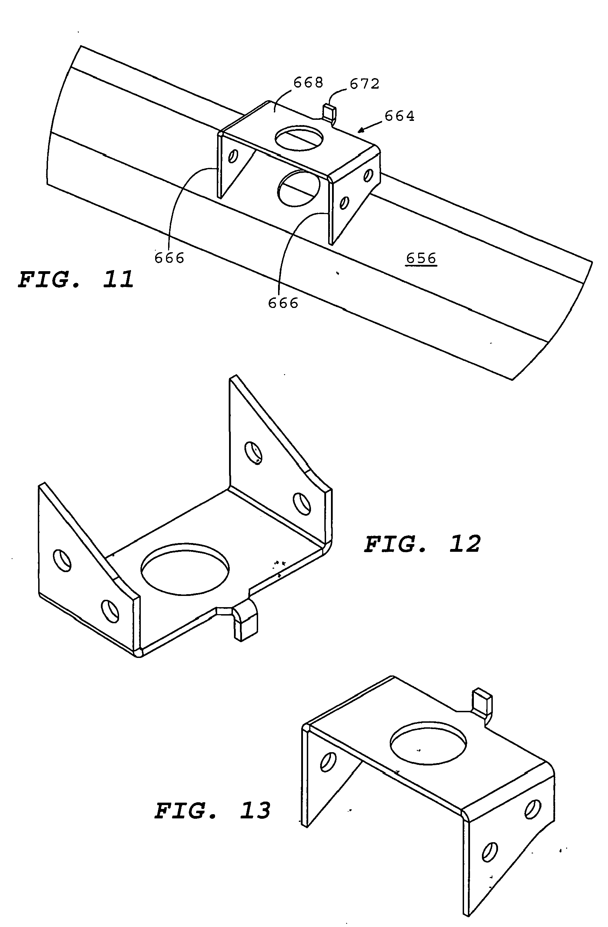

For additional convenience, in some aspects, an adjustable interface mounting assembly 660 can be coupled to the support surface 656 of the pillars 618. The adjustable interface mounting assembly 660 can be configured to allow the interface 652 to pivot or tilt with respect to a horizontal axis and swivel about the vertical axis if desired. As illustrated in Figs. 10-13, a flange base 664 can be coupled to the pillars 618 or, more specifically, the support surface 656 of the pillars 618. Although the flange base 664 can have a variety of shapes, the illustrated flange base 664 has two upright supports 666 extending upward from the support surface 656 and a body 668 extending between the two supports 666. An aperture is located in the body 668. The aperture allows a swivel pivot or rod 670 to extend between the body 668 and the interface 652 or other swivelable member. A projection 672 can also extend from the body 668. The projection 672 can be used as a stop to prevent swiveling beyond a predetermined limit.

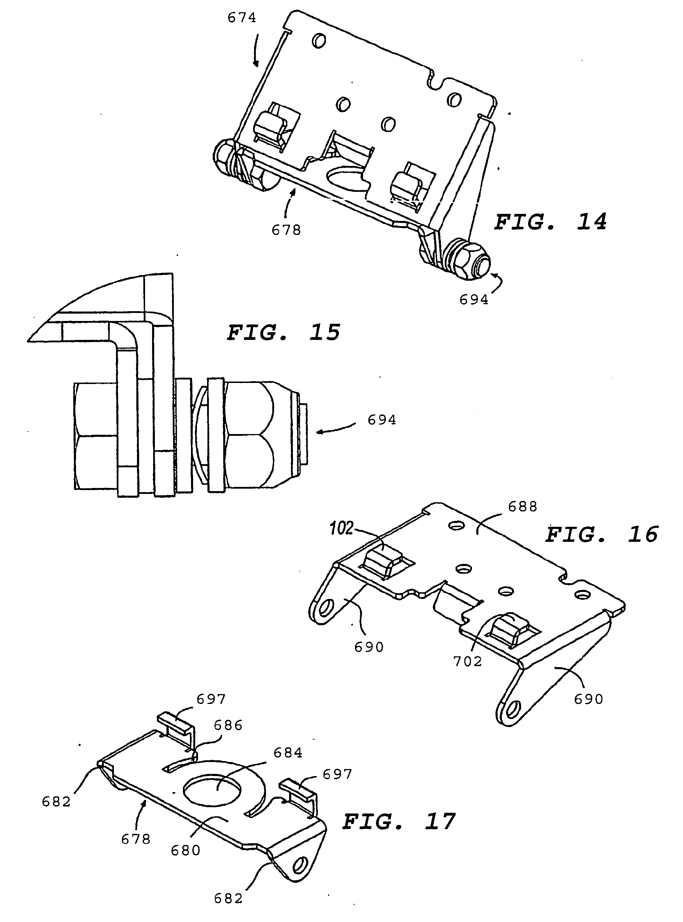

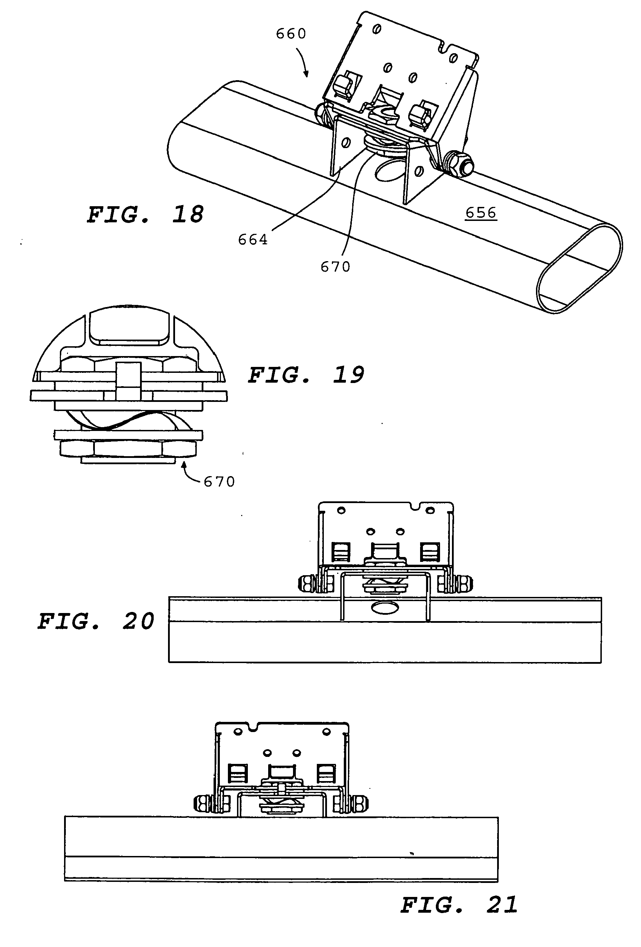

As illustrated in Figs. 14-21, a tilt mechanism 674 can be coupled to the flange base 664 via a swivel pivot (not shown), such as a rod, tube, threaded fastener, etc. The tilt mechanism 674 has a swivel member 678 having a main body portion 680 and ears 682 angled with respect to the main body portion 680. The main body portion 680 has an aperture 684 that also receives the swivel pivot to allow relative movement between the flange base 664 and the tilt mechanism 674. A curved groove 686 is centered about the aperture 680. The groove 686 is positioned and sized to accept the projection 672 from the flange base 664. The projection 672 can travel in this groove 686 as the tilt mechanism 674 is swiveled with respect to the flange mechanism 674. The relative movement is limited by the ends of the groove 686. Thus, when the projection 672 hits the end of the groove 686, the tilt mechanism 674 cannot swivel any further in that direction. The illustrated groove 686 allows the tilt mechanism 674 to swivel about 90 degrees in each direction. In other embodiments, the tilt mechanism 674 may swivel more or less.

A pivot platform 688 is coupled to the ears 682 of the swivel member 678. More specifically, the pivot platform 688 has two ears 690 that are angled with respect to the platform 688. These ears 690 align with the ears 682 of the swivel member 678. One or more pivots 694, such as threaded fasteners, rods, rivets, etc., extend between the ears 682 on the swivel member 678 and the ears 690 on the platform 688. This arrangement allows the platform 688 to be pivoted with respect to the swivel member 678. As illustrated in Fig. 17, the swivel member 678 (or the platform 688) can be equipped with one or more tabs to limit the amount of relative movement between the platform 688 and the swivel member 678.

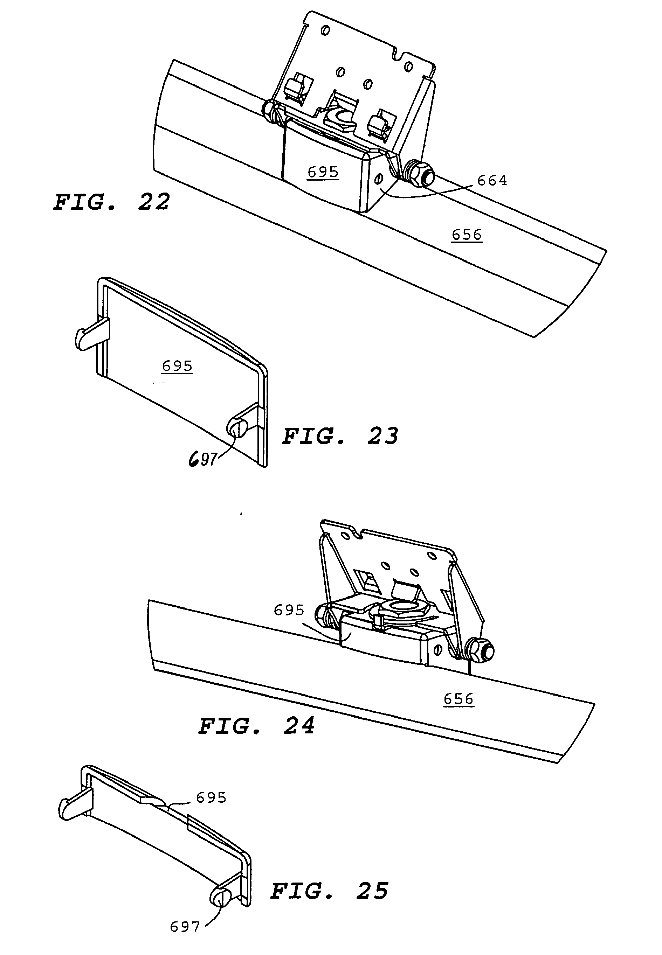

Once the swivel member 678 is coupled to the flange base 664 via the swivel pivot, the swivel pivot can be hidden with a pair of panels 695. As illustrated in Figs. 22-25, the panels 695 can be sized to abut the edges of the flange base 664 and cover the opening created by the U-shape of the flange base 664. Although these panels 695 can be attached in a variety of ways, the illustrated panels have projecting members 697 that form an interference fit with an aperture in the upright supports 666 of the flange base 664.

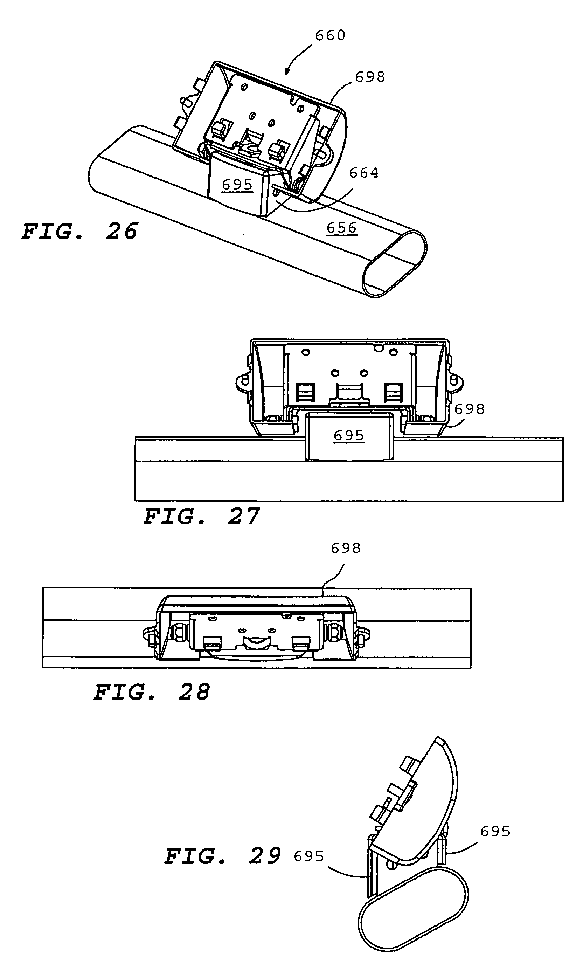

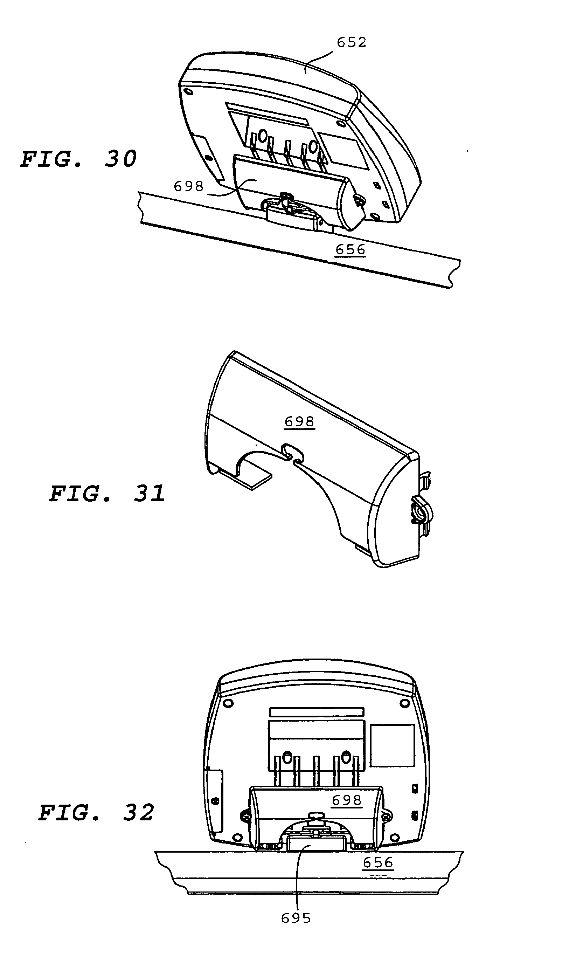

As illustrated in Figs. 26-32, the hardware of the tilt mechanism 674 may be covered or enclosed by a cover 698. The cover 698 can, for example, improve the aesthetics of the scale assembly 600 by hiding the hardware, eliminate exposed pinch points, etc. The cover 698 can be attached to the tilt mechanism 674 or can be coupled to the interface 652. The illustrated cover 698 has several protections that form an interference fit with the housing of the interface 652. However, other connections can be used. The connection between the housing of the interface 652 and the cover 698 will generally be sufficient to couple the interface 652 to the scale assembly 600. However, other connections can be used instead of or in addition to this coupling. For example, as illustrated in Figs. 14-21 projections 702 on the pivot platform can engage apertures on the housing of the interface 652 to provide for added security. Additionally, other fasteners can be used to connect the interface 652 to the scale assembly 600.

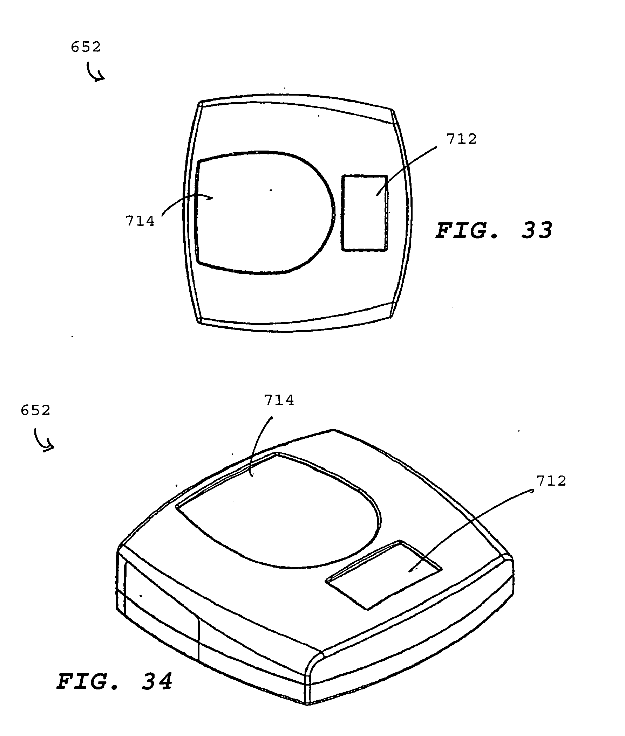

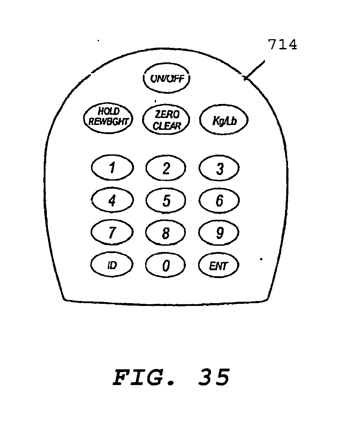

As shown in Figs. 33-35, the interface 652 includes a display 712 and an input (e.g., keypad 714). The keypad enables a user to input information and control the interface 652. The display 712 outputs visual information, such as measured height and weight data, and well as other information for the user to interact with and control the interface 652. The interface 652 also includes connectors for selectively coupling the weight measurement device 235 and height measurement device 240 thereto. For instance, the connectors are one of RS-232 ports, USB ports, or other data ports, and the weight measurement device 235 and height measurement device 240 include the appropriate output cables to connect to the interface 652. Thus, the interface 652 is selectively physically and electrically coupled to the scale assembly 600.

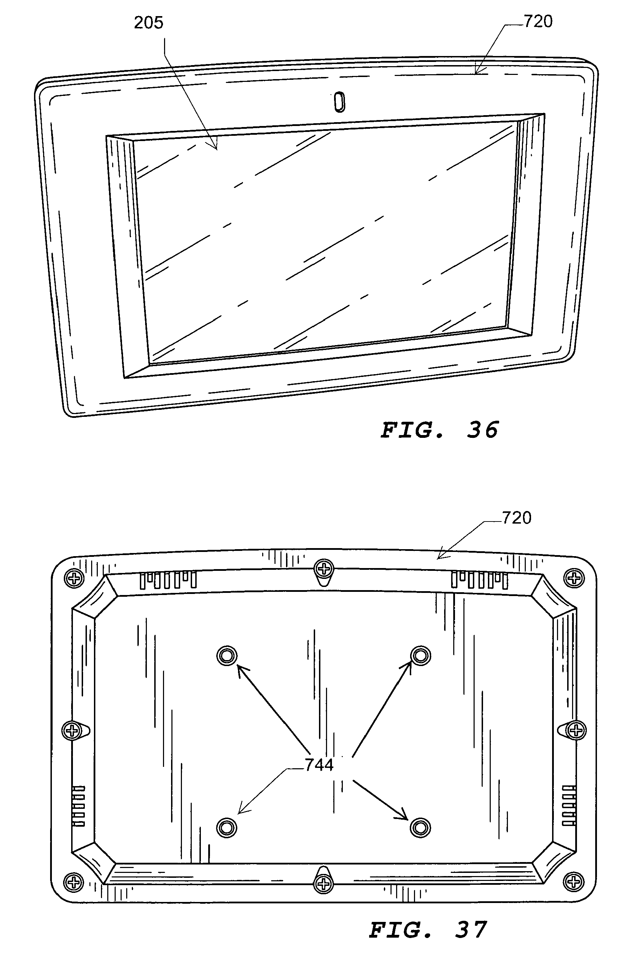

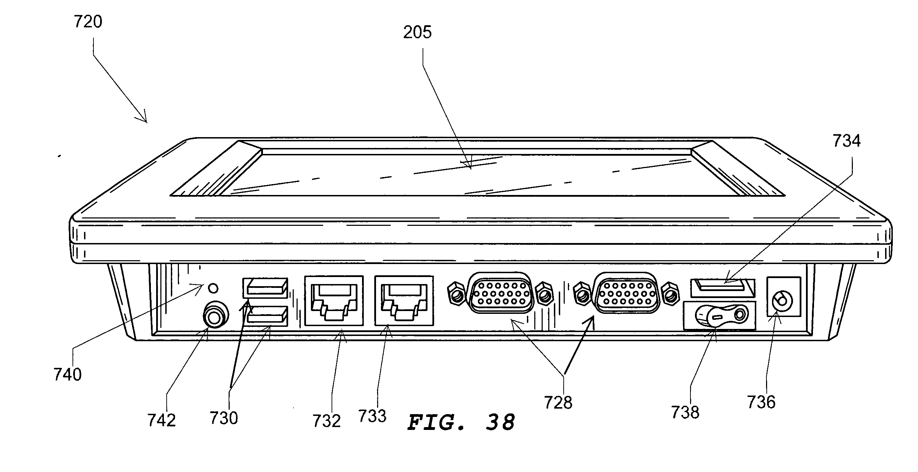

The selective coupling enables the swapping of the interface 652 for a new or replacement interface 652, in the case of malfunction, interfaces with upgraded features and physical components, or other interfaces. For instance, an adaptable interface 720 for the CTW 110 may be selectively coupled to the scale assembly 600. The adaptable interface 720 is depicted in Figs. 36-38 and includes the touch screen display 205, the memory 210, the processor 225, the input/output module 230, and the tracking module 245, as described above with respect to Fig. 2. As shown in Fig. 38, the illustrated adaptable interface 720 includes two RS-232 ports 728, two USB ports 730, one Ethernet ports 732, an RJ-45 port 733 for dedicated RS-485 serial communication, an HDMI port 734, a power cable input 736, an on/off switch 738, a reset button 740, and a line-out audio jack connector 742. Other combinations of inputs and outputs are used in some embodiments.

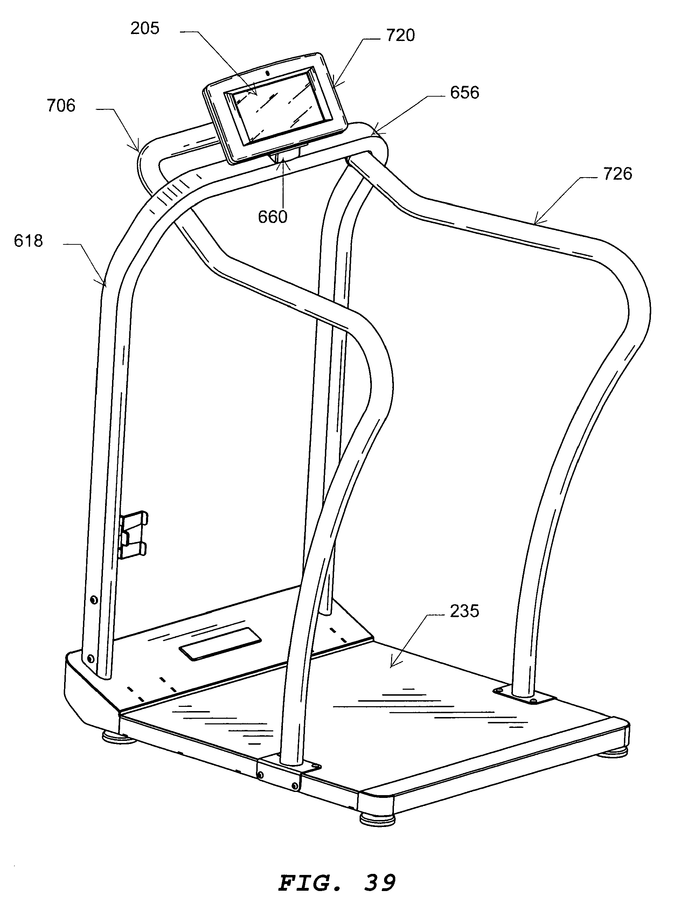

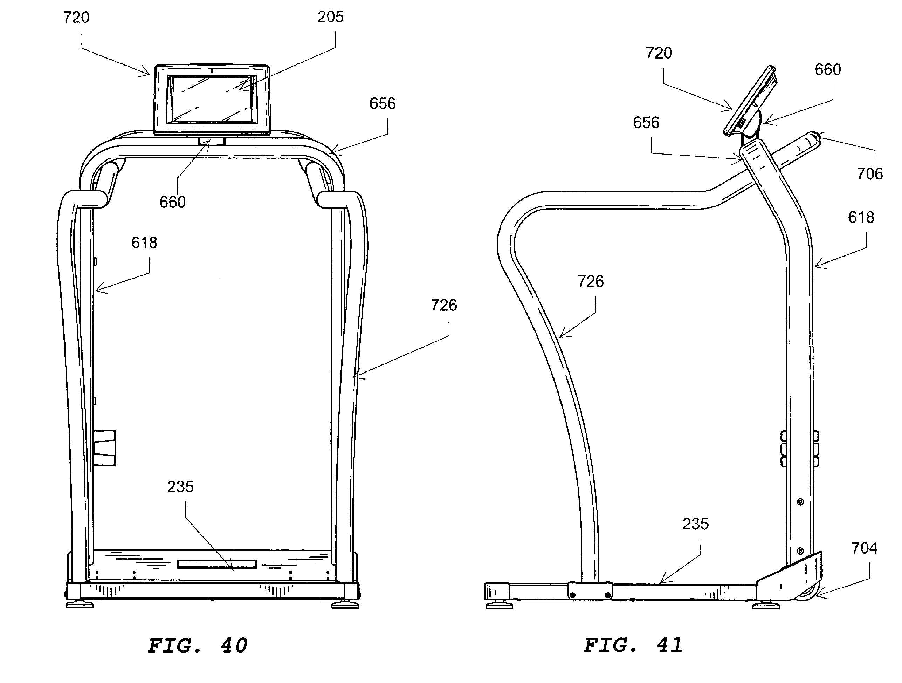

Similar to the interface 652, the adaptable interface 720 is mountable to a scale assembly (scale assembly 724) via mounting adapters 744 (see Figs. 37-39) and the adjustable interface mounting assembly 660 (see Figs. 39-41). The mounting adapters 744 are, for instance, threaded bore holes for receiving threaded fasteners to couple the adaptable interface 720 to the adjustable interface mounting assembly 660. Additionally, the interface 720 includes connectors (as shown in Fig. 38) for selectively coupling the weight measurement device 235, the height measurement device 240 and/or other devices thereto. The height measurement device 240 includes the appropriate output cables to connect to the adaptable interface 720.

In other constructions, different connecting structure may be provided to removably couple the interface(s) 652, 720 to the scale (e.g., scale 600, 724). For example, the alternative connecting structure may include quick-connect structure (e.g., movable projection(s) on one component engageable in recess(es) on the other, clamping devices, etc.) to releasably retain the interface 652, 720 on the scale. Such connecting structure may enable the interface 652, 720 to be more easily removed from the scale (e.g., to re-position the interface 652, 720 (e.g., on a surface near the scale), to use the interface 652, 720 for another operation away from the scale (e.g., with another device or as a stand alone device), etc.).

The illustrated scale assembly 724 differs from scale assembly 600 in that additional arms 726 are provided; however, each includes the adjustable interface mounting assembly 660. Thus, both the interface 652 and the adaptable interface 720 are selectively physically and electrically coupled to the scale assembly 600 and to the scale assembly 724 via the adjustable interface mounting assembly 660.

The selective coupling may also provide manufacturing benefits in the sense that a single physical structure (e.g., base 614, pillars 614, weight measurement device 235, and components other than the interface 652 and the adaptable interface 720) is able to be used for various interface models. For instance, the CTW 110 includes the adaptable interface 720 while a scale model with less functionality includes the interface 652. Another model may include more or fewer features as embodied in a different head used in place of the interface 652 or adaptable interface 720.

Each interchangeable interface (i.e., head) includes the physical ability to be mounted to the adjustable interface mounting assembly 660 and the necessary input/output ports to be coupled to the weight, height and/or other measurement devices. Furthermore, as shown, the adjustable interface mounting assembly 660 can be coupled to multiple different scale assemblies (e.g., scale assembly 600 and scale assembly 724). This interchangeability enables a modular system with multiple unique combinations of heads and scale assemblies to meet a user's particular needs. In some embodiments, the adjustable interface mounting assembly 660 is coupled to a scale assembly with a center post arrangement (not shown), rather than a scale assembly with two pillars 618 as provided in the scale assemblies 600 and 724.

Thus, the invention may provide, among other things, a reconfigurable CTW with drivers that enable the CTW to interface with different EHR systems. It should be understood that the CTW 110 may be used in a personal application, such as in a user's home, or in a commercial application, such as in a doctor's office, other health/medical facility, health club/gym, etc. In such applications, the CTW 110 may be reconfigurable to interface with the EHR system of the user's primary care physician, other health care provider, insurer, etc., or with another record system (e.g., a personal training records system used by a user, a health club/gym, etc.).

It should also be understood that the CTW 110 may be used to measure characteristic(s) of a subject other than a human subject, such as an animal subject or an inanimate subject (e.g., a package). The CTW 110 may be reconfigurable with a driver to interface with a record system associated with such a non-human subject.

One or more independent features and independent advantages of the invention may be set forth in the claims.

## Claims

1. A medical device comprising:
a memory;
a display;
a measurement component operable to determine a measured characteristic of a subject and to output measurement data indicative of the measured characteristic, the measured characteristic including one of a weight of the subject and a height of the subject; and
a processor coupled to the memory and to the display, the processor being operable to receive measurement data from the measurement component, output the measurement data to the display to cause the display to depict the measurement data, and store the measurement data in the memory, the processor including a reconfigurable driver module for communicating with external records databases, the reconfigurable driver module including a first driver, a second driver, a first communication configuration, and a second communication configuration, the first driver configuring the reconfigurable driver module into the first communication configuration to communicate with a first external records database, the second driver configuring the reconfigurable driver module into the second communication configuration to communicate with a second external records database, the second external records database having a different communication protocol than the first external records database.

2. The medical device of claim 1, wherein the first driver is obtained from a remote device via a network connection between the medical device and the remote device.

3. The medical device of claim 1, further comprising:
a user input device and web browsing software operable to be displayed on the display and manipulated via the user input device, the first driver being obtained from a website using the web browsing software; and/or
a network connection with a remote device, the medical device receiving diagnostic communications from the remote device and, in response, outputting diagnostic information to the remote device indicating whether the medical device is functioning; and/or a frame supporting the measurement component, and a head supported by the frame, the head including the memory, the display and the processor, the head being adjustably supported by the frame to adjust a position of the display.

4. The medical device of any preceding claim, wherein the processor communicates with the first external records database using the first driver to store the measurement data in the first external records database and communicates with the second external records database using the second driver to store the measurement data in the second external records database.

5. The medical device of any preceding claim, wherein, in response to receiving a command to store the measurement data in a third external records database, the processor
determines that a third driver is needed to communicate with the third external records database,
obtains the third driver from a remote device over a network, and
communicates the measurement data to the third external records database using the third driver.

6. The medical device of any preceding claim, wherein the measurement component includes one of a scale to measure the weight of the subject and a height rod to measure the height of the subject.

7. A method of communication between a medical device and remote databases, the medical device including a measurement component and a processor, the method comprising:
receiving, by the processor, measurement data from the measurement component, the measurement data indicating at least one of a weight of a subject and a height of a subject;
receiving a first driver and a second driver by the processor from one or more remote devices over a network, the first driver defining a first communications protocol for communicating with a first remote database and the second driver defining a second communications protocol for communicating with a second remote database;
receiving a command to store the measurement data in the first remote database; and communicating the measurement data to the first remote database for storage using the first driver.

8. The method of claim 7, further comprising:
receiving a second command to store the measurement data in the second remote database and communicating the measurement data to the second remote database for storage using the second driver; and/or
receiving a command to communicate with a third database using a third communications protocol, determining that the processor does not have a third driver for communicating with the third database using the third communications protocol, obtaining the third driver from the one or more remote devices over the network, and communicating the measurement data to the third remote database for storage using the third driver.

9. The method of claim 7 or 8, wherein communicating the measurement data to the first remote database for storage using the first driver includes
selecting the first driver,
configuring a driver module with the first driver,
receiving, by the driver module, a first communication including the measurement data from the processor,
translating, by the driver module, the first communication to satisfy the first communications protocol, and
sending the translated first communication to the first records database.

10. The method of claim 8, wherein obtaining the third driver includes:
downloading the third driver from a website using a web browser program executed by the processor and displayed on a display coupled to the processor; or
sending a request for the third driver to the one or more remote devices over the network, receiving the third driver over the network from the one or more remote devices.

11. A method of communication between a medical device and remote databases, the medical device including a measurement component and a processor, the method comprising:
receiving, by the processor, measurement data from the measurement component, the measurement data indicating at least one of a weight of a subject and a height of a subject;
receiving a command to store the measurement data in a first remote database;
determining that a first driver is an appropriate driver for communicating with the first remote database;
determining that the processor does not include the first driver;
obtaining the first driver from one or more remote devices over a network; and communicating the measurement data to the first remote database for storage using the first driver.

12. The method of claim 7 or 11, wherein the measurement data is associated with a patient, and wherein communicating includes communicating a patient identifier of the patient with the measurement data to the first remote database using the first driver, and the method optionally further comprises receiving, by the processor, new measurement data associated with the patient identifier from the first remote database using the first driver, storing the new measurement data in a local memory, and associating the stored new measurement data with the patient identifier.

13. The method of claim 11, further comprising:
receiving a second command to store the measurement data in a second remote database;
determining that a second driver is appropriate for communicating with the second remote database;
determining that the processor does not include the second driver;
obtaining the second driver from the one or more remote devices over a network;
communicating the measurement data to the second remote database for storage using the second driver.

14. A method of assembling a medical device, the method comprising:
providing a frame having a lower portion and an upper portion, a measurement component coupled to the frame and operable to determine one of a weight of a subject and a height of a subject, the measurement component being operable to output a signal representative of the one of the determined weight and the determined height, and an adjustable interface mounting assembly coupled to the upper portion of the frame;
providing a first interface head including a first display, a first input/output port for selectively coupling the first interface head to the measurement component, and a first mounting adapter for selectively coupling the first interface head to the adjustable interface mounting assembly, the first interface head being operable to receive measurement data from the measurement component and to display a value corresponding to the received measurement data; providing an adaptable, second interface head including a second display, a second input/output port for selectively coupling the second interface head to the measurement component, and a second mounting adapter for selectively coupling the second adjustable interface head to the adjustable interface mounting assembly, the second adjustable interface head being operable to receive measurement data from the measurement component and to display a value corresponding to the received measurement data, a memory, and a processor coupled to the memory and to the second display and including a reconfigurable driver module for communicating with external records databases, the reconfigurable driver module including a first driver, a second driver, a first communication configuration, and a second communication configuration, the first driver configuring the reconfigurable driver module into the first configuration to communicate with a first external records database and the second driver configuring the reconfigurable driver module into the second configuration to communicate with a second external records database, the second external records database having a different communication protocol than the first external records database;
selecting one of the first interface head and the second interface head; and coupling the one of the first interface head and the second interface head to the measurement component and to the adjustable interface mounting assembly.

15. An adaptable head assembly for a medical device, the medical device including a frame having a lower portion and an upper portion, a measurement component coupled to the frame and operable to determine one of a weight of a subject and a height of a subject, the measurement component being operable to output a signal representative of the one of the determined weight and the determined height, and an adjustable interface mounting assembly coupled to the upper portion of the frame, the head assembly comprising:
a housing including a mounting portion connectable to the mounting assembly to support the head on the frame;
a memory supported by the housing;
a display supported by the housing, the housing being adjustably supportable on the frame to adjust a position of the display;
an input/output port for selectively coupling the head assembly to the measurement component; and
a processor coupled to the memory, to the display and to the input/output port, the processor being operable to receive measurement data from the measurement component, output the measurement data to the display to cause the display to depict the measurement data, and store the measurement data in the memory, the processor including a reconfigurable driver module for communicating with external records databases, the reconfigurable driver module including a first driver, a second driver, a first communication configuration, and a second communication configuration, the first driver configuring the reconfigurable driver module into the first communication configuration to communicate with a first external records database, the second driver configuring the reconfigurable driver module into the second communication configuration to communicate with a second external records database, the second external records database having a different communication protocol than the first external records database, the reconfigurable driver module being operable to obtain at least one of the first driver and the second driver from a remote device over a network.
